# EUROPÄISCHE PATENTSCHRIFT

(11) **EP 3 757 940 B1**
(45) Veröffentlichungstag und Bekanntmachung des Hinweises auf die Patenterteilung: **16.04.2025**
(21) Anmeldenummer: 19182647.8
(22) Anmeldetag: 26.06.2019
(51) Int. Cl.: G06T 7/246

(54) **ERMITTLUNG EINER PATIENTENBEWEGUNG WÄHREND EINER MEDIZINISCHEN BILDGEBUNGSMESSUNG**
DETERMINATION OF A PATIENT'S MOVEMENT DURING A MEDICAL IMAGING MEASUREMENT
DÉTERMINATION D'UN MOUVEMENT DE PATIENT LORS D'UNE MESURE D'IMAGERIE MÉDICALE

(43) Veröffentlichungstag der Anmeldung: 30.12.2020
(73) Patentinhaber: Siemens Healthineers AG, 91301 Forchheim (DE)
(72) Erfinder: Kirsch, Rainer, 91083 Baiersdorf (DE)
(74) Vertreter: Siemens Healthineers Patent Attorneys

(56) Entgegenhaltungen:
- EP-A1- 3 477 583
- DE-A1- 102012 216 292

## Beschreibung

Die Erfindung betrifft ein Verfahren zu Ermittlung einer Patientenbewegung während einer medizinischen Bildgebungsmessung mit einer Bildgebungsvorrichtung, computerimplementierte Verfahren zum Bereitstellen trainierter Funktionen, eine Bildgebungsvorrichtung und ein Computerprogrammprodukt.

Bewegungen eines Patienten während einer medizinischen Bildgebungsmessung, beispielsweise bei einer Magnetresonanzbildgebung (engl. magnetic resonance imaging, MRI) oder einer Computertomographie (CT), können aus verschiedenen Gründen von Interesse sein. Insbesondere können Patientenbewegungen zu Bewegungsartefakten in resultierenden Abbildungen des Patienten führen. Bei Kenntnis der Patientenbewegungen können diese reduziert werden.

Die Druckschrift DE 10 2012 216292 A1 offenbart ein Verfahren zu einem Bestimmen einer Bewegung eines Patienten während einer Magnetresonanzuntersuchung mit Hilfe eines in einer in eine Kopfspulenvorrichtung angeordneten Bewegungssensoreinheit. Die Druckschrift EP 3 477 583 A1 offenbart eine Deep-Learning-basierte Verarbeitung von Bewegungsartefakten in Magnetresonanztomographie-Daten.

Als Aufgabe kann angesehen werden, Patientenbewegung während einer medizinischen Bildgebungsmessung zuverlässig und/oder schnell zu ermitteln.

Die Aufgabe wird durch die Merkmale der unabhängigen Ansprüche gelöst. Vorteilhafte Ausgestaltungen sind in den abhängigen Ansprüchen beschrieben.

Demnach wird ein computerimplementiertes Verfahren zu Ermittlung einer Patientenbewegung während einer medizinischen Bildgebungsmessung mit einer Bildgebungsvorrichtung vorgeschlagen. Dabei werden Referenz-Bilddaten eines Körperbereichs, z.B. eines Kopfbereichs, eines Patienten erfasst und mit Hilfe der Referenz-Bilddaten die Patientenbewegung ermittelt. Die Erfassung der Referenz-Bilddaten kann insbesondere mit einer, insbesondere optischen, Kamera erfolgen.

Vorzugsweise werden während der medizinischen Bildgebungsmessung medizinische Bildgebungs-Daten erfasst, die insbesondere geeignet sind, daraus diagnostische Abbildungen des Patienten zu erzeugen. Vorzugsweise wird anhand der medizinischen Bildgebungs-Daten und der ermittelten Patientenbewegung zumindest eine Abbildung des Patienten erstellt. Solche medizinischen Bildgebungs-Daten können beispielsweise Magnetresonanzsignale sein, aus denen Magnetresonanzabbildungen rekonstruiert werden können. Medizinische Bildgebungs-Daten können anhand der ermittelten Patientenbewegung korrigiert werden. Die Referenz-Bilddaten sind vorzugsweise optische Daten, d.h. die werden optisch erfasst, also mittels optischen Lichts.

Die Bildgebungsvorrichtung ist vorzugsweise eine Magnetresonanzvorrichtung. Magnetresonanzmessungen dauern vergleichsweise lange, weshalb sich hier auftretende Patientenbewegungen besonders stark auf Magnetresonanzabbildungen auswirken können. Bei Kenntnis der Patientenbewegungen können diese vorteilhafterweise wirkungsvoll kompensiert werden.

Beispielsweise kann eine laufende Bildgebungsmessung, insbesondere eine Sequenz einer Magnetresonanzmessung, prospektiv anhand der ermittelten Patientenbewegungen angepasst werden. Zusätzlich oder alternativ können gemessene medizinische Bildgebungs-Daten, beispielsweise Magnetresonanzsignale, retrospektiv anhand der ermittelten Patientenbewegungen korrigiert werden.

Bei den Patienten kann es sich insbesondere um einen menschlichen oder tierischen Patienten handeln. Patientenbewegungen können beispielsweise Bewegungen im Bereich des Kopfes, insbesondere des Gesichts, des Patienten sein.

Die Referenz-Bilddaten umfassen dreidimensionale Daten, insbesondere Tiefenbilder, des Köperbereichs des Patienten, beispielsweise seines Gesichts.

Es ist vorgesehen, dass anhand der Referenz-Bilddaten ein Referenz-Modell des erfassten Körperbereichs des Patienten erstellt wird. Ferner werden Patienten-Bilddaten eines Teils des Körperbereichs des Patienten, für den Referenz-Bilddaten erfasst wurden, während der medizinischen Bildgebungsmessung erfasst, d.h. die erfassten Patienten-Bilddaten bilden einen Teil des durch die Referenz-Bilddaten erfassten Körperbereichs des Patienten ab. Dieser Teil umfasst vorzugsweise nicht den gesamten durch die Referenz-Bilddaten erfassten Körperbereich des Patienten. Aus den Patienten-Bilddaten werden Anpassungsdaten erstellt. Durch Anpassen der Anpassungsdaten an das Referenz-Modell wird die Patientenbewegung ermittelt. Vorteilhafterweise wird durch das Anpassen der Anpassungsdaten an das Referenz-Modell ein Verhalten, insbesondere eine Bewegung, des durch die Patienten-Bilddaten nicht erfassten Körperbereichs des Patienten ermittelt.

Vorzugsweise erfolgt die Erfassung der Referenz-Bilddaten des Körperbereichs des Patienten vor der Bildgebungsmessung. Die Erfassung der Referenz-Bilddaten kann dabei insbesondere durch eine, insbesondere optische, Kamera erfolgen, die unabhängig von einer Aufnahmeeinheit der Bildgebungsvorrichtung ist. Eine solche Erfassung der Referenz-Bilddaten kann beispielsweise erfolgen, bevor der Patient in eine Bohrung einer Magnetresonanzvorrichtung gebracht wird, in der Magnetresonanzsignale erzeugt werden, die als medizinische Bildgebungs-Daten erfasst werden.

Es ist aber auch möglich, dass eine Kamera zur Erfassung der Referenz-Bilddaten an der Aufnahmeeinheit der Bildgebungsvorrichtung, beispielsweise an, insbesondere in, einer Bohrung (engl. bore) einer Magnetresonanzvorrichtung, angeordnet ist.

Erfindungsgemäß ist das Referenz-Modell ein dreidimensionales, insbesondere mathematisches, Modell. Beispielsweise umfasst das Referenz-Modell ein Gittermodell des Körperbereichs des Patienten, das durch räumlich verteilte Gitterpunkte beschrieben wird.

In das Referenz-Modell kann insbesondere anatomisches Vorwissen eingehen, das unabhängig von dem spezifischen Patienten ist, dessen Patientenbewegung hier ermittelt wird. Ein solches Vorwissen kann beispielsweise eine durchschnittliche und/oder über eine Vielzahl an Menschen gemittelte Gesichtsform sein. Vorzugsweise deckt das Referenz-Modell einen Bereich des Patientenkörpers ab, der größer ist als der Teil des Patientenkörpers, der während der medizinischen Bildgebungsmessung durch die Patienten-Bilddaten erfasst wird.

Die Erfassung der Patienten-Bilddaten kann insbesondere mit einer, insbesondere optischen, Kamera erfolgen. Die Patienten-Bilddaten sind also insbesondere optische Daten, die mittels optischen Lichts durch eine Kamera erfasst werden. Diese Kamera kann insbesondere dieselbe sein, mit der auch die Erfassung der Referenz-Bilddaten erfolgt. Sie kann aber auch eine andere Kamera sein. Vorzugsweise ist die Kamera zur Erfassung der Patienten-Bilddaten an der Aufnahmeeinheit der Bildgebungsvorrichtung, beispielsweise an, insbesondere in, einer Bohrung einer Magnetresonanzvorrichtung, angeordnet. Beispielsweise ist eine solche Kamera an der Innenseite der Bohrung über der Position angeordnet, in der üblicherweise der Kopf des Patienten gelagert wird.

Die Erfassung der Patienten-Bilddaten kann insbesondere kontinuierlich während der medizinischen Bildgebungsmessung erfolgen. Eine kontinuierliche Erfassung der Patienten-Bilddaten kann beispielsweise eine Messung sein, bei der mit einer Wiederholfrequenz von 10 bis 100 Hz, insbesondere 60 Hz, Abbildungen des Teils des Körperbereichs des Patienten erzeugt werden.

Vorzugsweise umfassen die Patienten-Bilddaten dreidimensionale Daten, insbesondere Tiefenbilder des Patienten, beispielsweise seines Gesichts. Aus dreidimensionale Patienten-Bilddaten können insbesondere mehrdimensionale, insbesondere dreidimensionale, Anpassungsdaten abgeleitet werden, die besonders effektiv an ein dreidimensionales Referenz-Modell gepasst werden können.

Das Anpassen der Anpassungsdaten an das Referenz-Modell kann beispielsweise ein Fitting oder eine Regression der Anpassungsdaten an das Referenz-Modell umfassen. Vorzugsweise wird bei dem Anpassen der Anpassungsdaten an das Referenz-Modell zumindest ein Teil des Körperbereichs des Patienten rekonstruiert, der durch die Patienten-Bilddaten nicht erfasst wurde.

Durch das Anpassen der Anpassungsdaten an das Referenz-Modell kann vorteilhafterweise die Patientenbewegung des gesamten Körperbereichs des Patienten ermittelt werden, obwohl nur ein Teil des Körperbereichs während der der medizinischen Bildgebungsmessung erfasst wird. Dies kann insbesondere dann von Vorteil sein, wenn dieser Körperbereich teilweise abgedeckt wird. Dadurch wird insbesondere der Körperbereich nur nur unvollständig, insbesondere mit Lücken, erfasst.

Beispielsweise wird während der Erfassung der Patienten-Bilddaten der Körperbereich des Patienten durch die Bildgebungsvorrichtung teilweise abgedeckt, so dass nur ein Teil des Körperbereichs während der der medizinischen Bildgebungsmessung durch die Patienten-Bilddaten erfasst wird.

Die Erstellung der Anpassungsdaten aus den Patienten-Bilddaten kann insbesondere eine direkte und/oder unveränderte Übernahme der Patienten-Bilddaten umfassen. Insbesondere können die Anpassungsdaten identisch mit den Patienten-Bilddaten sein. Alternativ ist möglich, dass die Anpassungsdaten gegenüber den Patienten-Bilddaten modifiziert sind.

Eine weitere Ausführungsform des Verfahrens zur Ermittlung einer Patientenbewegung sieht vor, dass eine Geometrie-Information eines Moduls der Bildgebungsvorrichtung bereitgestellt wird. Dabei deckt das Modul den Körperbereich des Patienten während der Erfassung der Patienten-Bilddaten teilweise, insbesondere nicht vollständig, ab. Die Erstellung von Anpassungsdaten erfolgt dabei mit Hilfe der Geometrie-Information. Die Patientenbewegung kann dann vorteilhafterweise durch eine Berücksichtigung der Geometrie-Information des Moduls genauer erfolgen.

Das Modul der Bildgebungsvorrichtung kann insbesondere ein Teil der Bildgebungsvorrichtung sein, der während der Erfassung der Patienten-Bilddaten den Körperbereich des Patienten teilweise abdeckt. Das Modul der Bildgebungsvorrichtung kann beispielsweise eine Lokalspule (engl. local coil) sein. Eine Lokalspule umfasst typischerweise eine oder mehrere Antennen, die ausgebildet sind, hochfrequente (HF, engl. RF) Signale zu senden und/oder zu empfangen. Lokalspulen werde typischerweise möglichst nahe an dem Patienten angeordnet, um ein hohes Signal-RauschVerhältnis empfangener Magnetresonanzsignale zu erreichen.

Vorzugsweise ist der erfasste Körperbereich des Patienten ein Kopfbereich des Patienten, und die die Lokalspule ist eine Kopfspule. Vorzugsweise weist die Kopfspule eine oder mehrere Durchbrüche auf, so dass nur ein Teil des Kopfes des Patienten abgedeckt wird, wenn die Kopfspule am Patienten angeordnet ist. Beispielsweise umfasst die Kopfspule ein (anteriores) Oberteil, das solche Durchbrüche aufweist.

Eine weitere Ausführungsform des Verfahrens zur Ermittlung einer Patientenbewegung sieht vor, dass die Erstellung der Anpassungsdaten aus den Patienten-Bilddaten eine Erstellung von Differenz-Bilddaten aus der Geometrie-Information des Moduls und den Patienten-Bilddaten umfasst. Vorteilhafterweise werden die Differenz-Bilddaten als Anpassungsdaten verwendet. Vorzugsweise umfassen die Anpassungsdaten lediglich Segmente der Patienten-Bilddaten, die während der Erfassung der Patienten-Bilddaten nicht abgedeckt wurden. Beispielsweise werden die Geometrie-Information von den Patienten-Bilddaten subtrahiert. Vorzugsweise werden die Segmente der Patienten-Bilddaten, die der Geometrie-Information des Moduls entsprechen, identifiziert und beispielsweise aus den Patienten-Bilddaten entfernt.

Vorteilhafterweise kann anhand der Differenz-Bilddaten die Anpassung der Patienten-Bilddaten an das Referenz-Modell und/oder die Ermittlung der Patientenbewegung genauer und/oder zuverlässiger erfolgen, da die patientenunabhängigen, insbesondere unbeweglichen, Teile der Patienten-Bilddaten entfernt wurden.

Eine weitere Ausführungsform des Verfahrens sieht vor, dass die Erstellung der Differenz-Bilddaten aus der Geometrie-Information des Moduls und den Patienten-Bilddaten ein Anwenden einer durch maschinelles Lernen trainierten Funktion auf die Geometrie-Information des Moduls und die Patienten-Bilddaten umfasst, wobei Differenz-Bilddaten erzeugt werden.

Vorzugsweise wird das Anwenden der Funktion mit Hilfe einer Recheneinheit durchgeführt.

Die Geometrie-Information kann beispielsweise in einer Datenbank vorliegen, in der geometrische Daten, z.B. CAD-Daten, zu einem oder mehreren Modulen gespeichert sind. Im Rahmen der medizinischen Bildgebungsmessung kann das Modul angegeben werden, das während der medizinischen Bildgebungsmessung eingesetzt werden soll. Die Bereitstellung der Geometrie-Information des Moduls kann beispielsweise ein Abrufen dieser geometrischen Daten aus der Datenbank umfassen.

Vorzugsweise umfasst die Bereitstellung der Geometrie-Information des Moduls der Bildgebungsvorrichtung eine Erfassung von Modul-Bilddaten des Moduls der Bildgebungsvorrichtung. Die Erfassung der Modul-Bilddaten kann insbesondere mit einer, insbesondere optischen, Kamera erfolgen. Diese Kamera kann insbesondere dieselbe sein, mit der auch die Erfassung der Referenz-Bilddaten und/oder der Patienten-Bilddaten erfolgt. Sie kann aber auch eine andere Kamera sein.

Beispielsweise wird mit einer Kamera ein Oberteil einer Kopfspule als Modul-Bilddaten aufgenommen. Während der medizinischen Bildgebungsmessung wird das Oberteil der Kopfspule mitsamt der zwischen Durchbrüchen des Oberteils der Kopfspule sichtbaren Segmente des Patientengesichts als Patienten-Bilddaten aufgenommen. Durch Subtraktion der Modul-Bilddaten von den Patienten-Bilddaten und/oder Anwenden einer trainierten Funktion auf die Modul-Bilddaten und die Patienten-Bilddaten erhält man vorteilhafterweise vom Oberteil der Kopfspule bereinigte Differenz-Bilddaten. Vorteilhafterweise wird durch das Anpassen der Differenz-Bilddaten an das Referenz-Modell ein Verhalten, insbesondere eine Bewegung, des gesamten Gesichtsfelds des Patienten (einschließlich der durch das Oberteil der Kopfspule abgedeckten Teile) ermittelt.

Vorzugsweise werden die Modul-Bilddaten des Moduls aus derselben Perspektive aufgenommen wie die Patienten-Bilddaten des Köperbereichs des Patienten während der medizinischen Bildgebungsmessung. Dies ermöglicht vorteilhafterweise eine besonders einfache Erstellung von Differenz-Bilddaten.

Vorzugsweise erfolgt die Ermittlung der Patientenbewegung, insbesondere durch Anpassen der Anpassungsdaten an das Referenz-Modell, in Echtzeit, d.h. unmittelbar nach der Erfassung der jeweiligen Patienten-Bilddaten. Somit stehen die Patientenbewegungsinformationen sofort zur weiteren Verarbeitung zur Verfügung. Beispielsweise wird die ermittelte Patientenbewegung für eine funktionelle Magnetresonanzbildgebung und/oder zur Bewegungskorrektur verwendet.

Eine weitere Ausführungsform des Verfahrens sieht vor, dass die Ermittlung der Patientenbewegung durch Anpassen der Anpassungsdaten an das Referenz-Modell ein Anwenden einer durch maschinelles Lernen trainierten Funktion auf die Anpassungsdaten umfasst, wobei Patientenbewegungsdaten erzeugt werden. Vorzugsweise wird das Anwenden der Funktion mit Hilfe einer Recheneinheit durchgeführt.

Eine weitere Ausführungsform des Verfahrens sieht vor, dass der Körperbereich ein Gesicht des Patienten ist, wobei die Erfassung der Referenz-Bilddaten des Körperbereich des Patienten durch einer Miniaturkamera erfolgt, die in eine Kopfspule angeordnet ist.

Beispielsweise können mit Hilfe einer Minikamera in Echtzeit die Referenz-Bilddaten des Gesichtes aufgenommen werden, die Position und die Bewegung des Kopfes in Echtzeit ohne Anbringung externer Markierungen erkannt und insbesondere zur Bewegungskorrektur genutzt werden. Dabei sind die Referenz-Bilddaten vorzugsweise Tiefenbilder.

Dazu können vorteilhafterweise dreidimensionale Gesichtsverfolgungsalgorithmen, wie sie beispielsweise in Mobilfunkgeräten eingesetzt werden, verwendet werden. Dabei können insbesondere dreidimensionale Gitterbilder erstellt werden, um daraus die Patientenbewegung zu bestimmen. Vorzugweise ist während der medizinischen Bildgebungsmessung am Patienten, insbesondere am Kopf des Patienten keine externe Markierung angebracht. Eine solche externe Markierung kann insbesondere eine Markierung sein, deren Erfassung zur Ermittlung einer Patientenbewegung geeignet ist. Insbesondere kann solche externe Markierung eine Markierung sein, deren Erfassung zur Ermittlung einer Bewegung des Köperteils, an dem die Markierung angebracht ist, geeignet ist. Eine externe Markierung kann beispielsweise eine Landmarke sein.

Vorzugsweise erfolgt die Ermittlung der Patientenbewegung innerhalb einer vorbestimmten Zeitspanne, zum Beispiel in einem festen Zeitraster, bestimmt. Diese vorbestimmte Zeitspanne beträgt vorzugsweise weniger als 10 Sekunden, insbesondere weniger als 1 Sekunde, insbeosndere weniger als 0,1 Sekunden.

Ferner wird ein computerimplementiertes Verfahren zum Bereitstellen einer trainierten Funktion zur Erzeugung von Differenz-Bilddaten vorgeschlagen. Dieses Verfahren umfasst ein Empfangen von Trainings-Modul-Bilddaten und Trainings-Patienten-Bilddaten und ein Empfangen von Trainings-Differenz-Bilddaten. Dabei sind die Trainings-Differenz-Bilddaten mit den Trainings-Modul-Bilddaten und Trainings-Patienten-Bilddaten verknüpft. Zudem umfasst das Verfahren ein Trainieren einer Funktion basierend auf den Trainings-Modul-Bilddaten, den Trainings-Patienten-Bilddaten und den Trainings-Differenz-Bilddaten und ein Bereitstellen der trainierten Funktion zur Erzeugung von Differenz-Bilddaten.

Die Trainings-Modul-Bilddaten sind beispielsweise Daten, die ein Modul einer Bildgebungsvorrichtung abbilden. Die Trainings-Patienten-Bilddaten sind beispielsweise Daten, die einen Bereich des Patienten abbilden.

Die trainierte Funktion basiert vorzugsweise auf einem neuronalen Netzwerk, insbesondere einem Convolutional Neural Network (CNN) oder einem Deep Convolutional Neural Network.

Insbesondere wird eine Trainingssystem vorgeschlagen, das eine erste Trainings-Schnittstelle zum Empfangen von Trainings-Modul-Bilddaten, eine zweite Schnittstelle zum Empfangen von Trainings-Patienten-Bilddaten, eine dritte Schnittstelle zum Empfangen von Trainings-Differenz-Bilddaten, eine Trainings-Berechnungseinheit zum Trainieren einer Funktion basierend auf den Trainings-Modul-Bilddaten, den Trainings-Patienten-Bilddaten und den Trainings-Trainings-Differenz-Bilddaten und eine vierte Trainings-Schnittstelle zum Bereitstellen der trainierten Funktion umfasst.

Ferner wird ein computerimplementiertes Verfahren zum Bereitstellen einer trainierten Funktion zur Erzeugung von Patientenbewegungsdaten vorgeschlagen. Dieses Verfahren umfasst ein Empfangen von Trainings-Anpassungsdaten und ein Empfangen von Trainings-Patientenbewegungsdaten. Dabei sind die Trainings-Patientenbewegungsdaten mit den Trainings-Anpassungsdaten verknüpft. Zudem umfasst das Verfahren ein Trainieren einer Funktion basierend auf den Trainings-Patientenbewegungsdaten und den Trainings-Anpassungsdaten und ein Bereitstellen der trainierten Funktion zur Erzeugung von Patientenbewegungsdaten.

Die Trainings-Anpassungsdaten sind beispielsweise Daten, die einen Bereich des Patienten nur unvollständig, insbesondere mit Lücken, abbilden. Die Trainings-Anpassungsdaten sind beispielsweise Daten, die einen Bereich des Patienten nur vollständig, insbesondere lückenlos, abbilden.

Insbesondere sind die Trainings-Anpassungsdaten und die Trainings-Patientenbewegungsdaten zeitabhängige Daten. Insbesondere bilden die Trainings-Anpassungsdaten und die Trainings-Patientenbewegungsdaten eine Bewegung des Patienten ab.

Die trainierte Funktion basiert vorzugsweise auf einem neuronalen Netzwerk, insbesondere einem Convolutional Neural Network (CNN) oder einem Deep Convolutional Neural Network.

Insbesondere wird ein Trainingssystem vorgeschlagen, das eine erste Trainings-Schnittstelle zum Empfangen von Trainings-Anpassungsdaten, eine zweite Schnittstelle zum Empfangen von Trainings-Patientenbewegungsdaten, eine Trainings-Berechnungseinheit zum Trainieren einer Funktion basierend auf den Trainings-Anpassungsdaten und den Trainings-Patientenbewegungsdaten und eine dritte Trainings-Schnittstelle zum Bereitstellen der trainierten Funktion umfasst.

Ferner wird eine Bildgebungsvorrichtung, insbesondere eine Magnetresonanzvorrichtung vorgeschlagen, die ausgebildet ist, ein vorab beschriebenes Verfahren zur Ermittlung einer Patientenbewegung während einer medizinischen Bildgebungsmessung mit der Bildgebungsvorrichtung auszuführen. Beispielsweise umfasst die Bildgebungsvorrichtung eine oder mehrere Kameras zur Erfassung von Referenz-Bilddaten und/oder Patienten-Bilddaten. Beispielsweise umfasst die Bildgebungsvorrichtung eine Systemsteuereinheit zur Ermittlung der Patientenbewegung und/oder zu einer Erstellung eines Referenz-Modells und/oder zu einer Erstellung von Anpassungsdaten mit Hilfe erfasster Daten. Beispielsweise umfasst die Bildgebungsvorrichtung ein oder mehrere Module, die ausgebildet sind, den Körperbereich des Patienten während der Erfassung von Patienten-Bilddaten teilweise abzudecken.

Ferner wird ein Computerprogrammprodukt vorgeschlagen, das ein Programm umfasst und direkt in einen Speicher einer programmierbaren Systemsteuereinheit einer Bildgebungsvorrichtung, insbesondere einer Magnetresonanzvorrichtung, ladbar ist und Programmmittel, z.B. Bibliotheken und Hilfsfunktionen, aufweist, um ein Verfahren zur Ermittlung einer Patientenbewegung während einer medizinischen Bildgebungsmessung mit der Bildgebungsvorrichtung auszuführen, wenn das Computerprogrammprodukt in der Systemsteuereinheit der Bildgebungsvorrichtung ausgeführt wird. Das Computerprogrammprogrammprodukt kann dabei eine Software mit einen Quellcode, der noch kompiliert und gebunden oder der nur interpretiert werden muss, oder einen ausführbaren Softwarecode umfassen, der zur Ausführung nur noch in die Systemsteuereinheit zu laden ist. Durch das Computerprogrammprodukt kann das besagte Verfahren schnell, identisch wiederholbar und robust ausgeführt werden. Das Computerprogrammprodukt ist so konfiguriert, dass es mittels der Systemsteuereinheit die erfindungsgemäßen Verfahrensschritte ausführen kann. Die Systemsteuereinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem Netzwerk oder Server hinterlegt, von wo es in den Prozessor einer lokalen Systemsteuereinheit geladen werden kann, der mit der Bildgebungsvorrichtung direkt verbunden oder als Teil der Bildgebungsvorrichtung ausgebildet sein kann.

Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Systemsteuereinheit einer Bildgebungsvorrichtung ein erfindungsgemäßes Verfahren durchführen. Beispiele für elektronische lesbare Datenträger sind eine DVD, ein Magnetband oder einen USB-Stick, auf welchem elektronisch lesbare Steuerinformationen, insbesondere Software, gespeichert ist. Wenn diese Steuerinformationen von dem Datenträger gelesen und in eine Systemsteuereinheit der Bildgebungsvorrichtung gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: eine Magnetresonanzvorrichtung mit einer Kopfspule und einer Kamera,
- Fig. 2: ein Blockdiagramm eines ersten Verfahrens zur Ermittlung einer Patientenbewegung während einer medizinischen Bildgebungsmessung mit einer Bildgebungsvorrichtung,
- Fig. 3: eine Kopfspule mit einer Minikamera,
- Fig. 4: ein Gesicht eines Patienten mit einem die Oberfläche des Gesichts beschreibenden Gitternetz,
- Fig. 5: ein Blockdiagramm eines zweiten Verfahrens zur Ermittlung einer Patientenbewegung während einer medizinischen Bildgebungsmessung mit einer Bildgebungsvorrichtung,
- Fig. 6: ein Gesicht eines Patienten, das teilweise von einer Kopfspule abgedeckt wird,
- Fig. 7: eine Kopfspule, die abdeckende und durchsichtige Bereiche aufweist,
- Fig. 8: ein Blockdiagramm eins computerimplementierten Verfahrens zum Bereitstellen einer trainierten Funktion zur Erzeugung von Differenz-Bilddaten,
- Fig. 9: ein Blockdiagramm eins computerimplementierten Verfahrens zum Bereitstellen einer trainierten Funktion zur Erzeugung von Patientenbewegungsdaten.

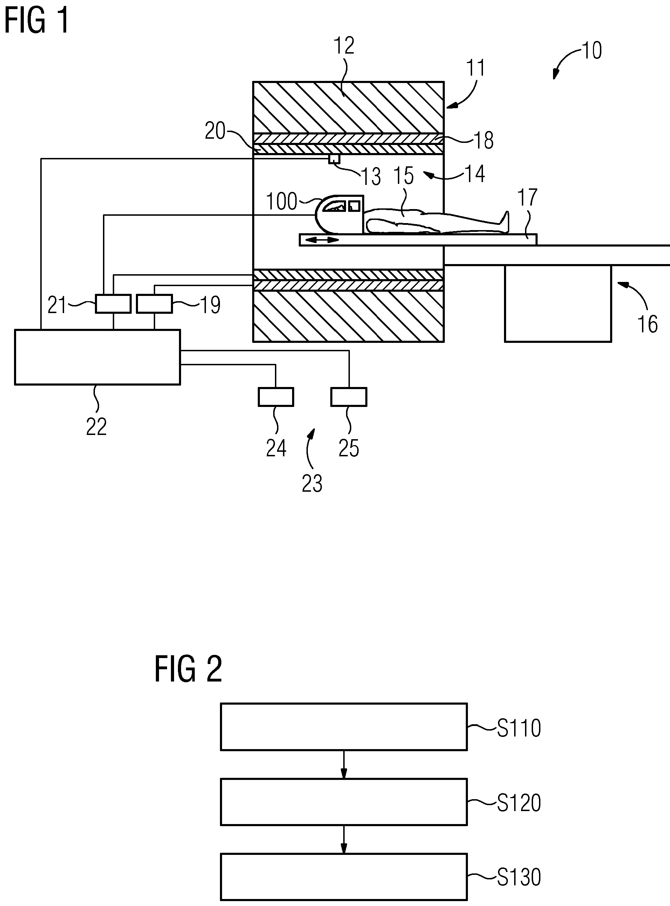

In Fig. 1 ist beispielhaft für eine Bildgebungsvorrichtung eine Magnetresonanzvorrichtung 10 schematisch dargestellt. Die Magnetresonanzvorrichtung 10 umfasst eine Magneteinheit 11, die einen Hauptmagneten 12 zu einem Erzeugen eines starken und insbesondere zeitlich konstanten Hauptmagnetfelds aufweist. Zudem umfasst die Magnetresonanzvorrichtung 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Die Hochfrequenzantenneneinheit 20 ist zu einer Anregung von Atomkernen, die sich in dem von dem Hauptmagneten 12 erzeugten Hauptmagnetfeld einstellt, ausgelegt. Die Hochfrequenzantenneneinheit 20 wird von einer Hochfrequenzantennensteuereinheit 21 der Magnetresonanzvorrichtung 10 gesteuert und strahlt Hochfrequenzpulse in einen Untersuchungsraum ein, der im Wesentlichen von einem Patientenaufnahmebereich 14 der Magnetresonanzvorrichtung 10 gebildet ist. Die Hochfrequenzantenneneinheit 20 ist weiterhin zum Empfang von Magnetresonanzsignalen ausgebildet. Ferner weist die Magnetresonanzvorrichtung ein Modul in Form einer Kopfspule 100 auf, die um den Kopf des Patienten 15 herum angeordnet ist. Die Kopfspule 100 ist eine Lokalspule, die ausgebildet ist, Hochfrequenzpulse auszusenden und/oder Magnetresonanzsignale zu empfangen.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert eine medizinische Bildgebungsmessung der Magnetresonanzvorrichtung 10, wie beispielsweise das Durchführen einer vorbestimmten bildgebenden Sequenz. Zudem umfasst die Systemsteuereinheit 22 eine nicht näher dargestellte Auswerteeinheit zu einer Auswertung von medizinischen Bildgebungs-Daten, die hier in Form von Magnetresonanzsignalen während der Magnetresonanzuntersuchung erfasst werden. Ferner umfasst die Systemsteuereinheit 22 einen Speicher, in das direkt ein Programm geladen werden kann, um insbesondere eines der nachfolgend beschriebenen Verfahren zur Ermittlung einer Patientenbewegung auszuführen, wenn das Programm in der Systemsteuereinheit 22 der Magnetresonanzvorrichtung 10 ausgeführt wird.

Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzabbildungen können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Die Magnetresonanzvorrichtung umfasst ferner eine Kamera 13, die an der Innenseite der Magneteinheit 11 angeordnet ist. Die Kamera 13 ist ausgebildet, Bilddaten zu erfassen und an die Systemsteuereinheit 22 zu übermitteln. Insbesondere kann die Kamera 13 Bilddaten aus dem Bereich des Kopfs des Patienten 15 aufnehmen. Die Kopfspule 100 deckt während der medizinischen Bildgebungsmessung einen Teil des Kopfes ab, so dass die Bilddaten teilweise die Kopfspule und teilweise den Kopf, insbesondere das Gesicht, des Patienten beschreiben.

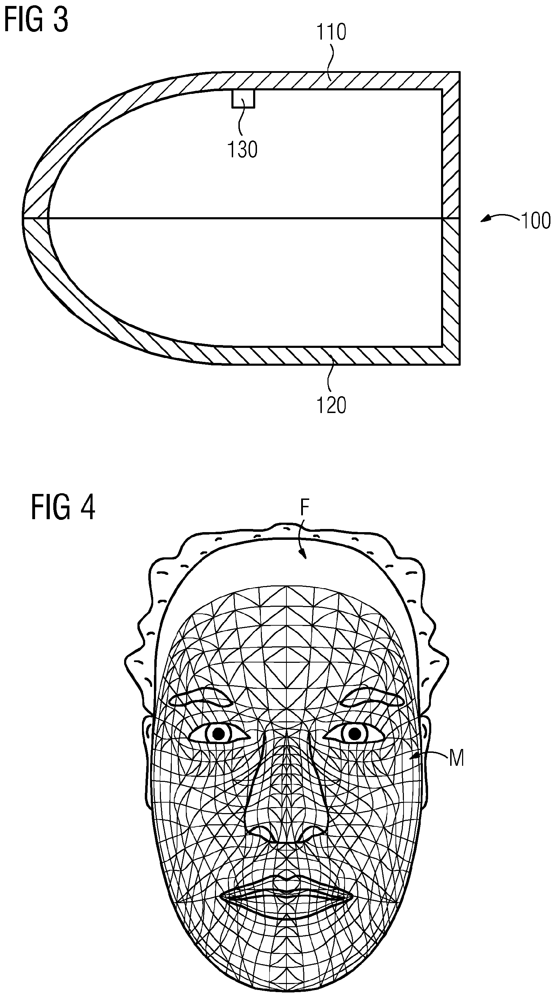

Die Magnetresonanzvorrichtung ist ausgebildet, ein Verfahren zur Ermittlung einer Patientenbewegung während einer medizinischen Bildgebungsmessung mit der Magnetresonanzvorrichtung auszuführen. Ein solches Verfahren ist beispielhaft in Fig. 2 und 3 dargestellt.

Dabei werden in S110 Referenz-Bilddaten eines Körperbereichs, hier des Kopfbereichs, des Patienten 15 erfasst.

In S120 wird die Patientenbewegung mit Hilfe der Referenz-Bilddaten ermittelt. Dabei ist beispielsweise der Körperbereich ein Gesicht des Patienten und die Erfassung der Referenz-Bilddaten des Körperbereich des Patienten erfolgt durch einer Miniaturkamera, die in einer Kopfspule angeordnet ist.

In S130 werden anhand der erfassten Magnetresonanzsignale und der ermittelten Bewegung des Kopfes eine oder mehrere Abbildung des Patienten in Form von Magnetresonanzabbildungen rekonstruiert.

In Fig. 3 ist eine Kopfspule 100 dargestellt, in die eine, insbesondere optische, Miniaturkamera 130 zur Erfassung von Bilddaten eines Gesichts eines Patienten 15 integriert ist. Sie umfasst hier ein Oberteil 110 und ein Unterteil 120, auf dem der Kopf des Patienten 15 gelagert ist. An der Innenseite des Oberteils ist die Miniaturkamera 130 angeordnet und verfügt über ein Sichtfeld, das ausreichend groß ist, um das Gesicht des Patienten 15 zu erfassen.

In Fig. 4 ist ein Gesicht F eines Patienten 15 dargestellt. Vorteilhafterweise können mit Hilfe der Miniaturkamera 130 in Echtzeit Tiefenbilder des Gesichts F aufgenommen werden. Aus den Tiefenbildern können beispielsweise ein dreidimensionales Gittermodell M des Gesichts F ermittelt werden. Aus den Tiefenbildern und/oder dem Gittermodell M kann die Position und/oder die Bewegung des Kopfes in Echtzeit erkannt werden. Dabei kann vorzugsweise auf eine Anbringung von Landmarken auf dem Gesicht des Patienten 15 verzichtet werden. Die erkannte Bewegung kann beispielsweise zur Bewegungskorrektur genutzt werden.

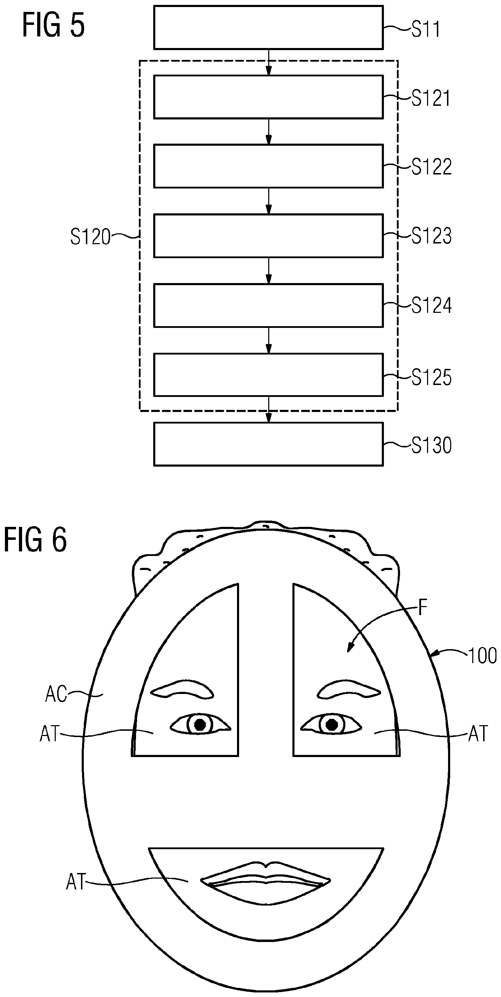

In Fig. 5 ist ein weiteres Ausführungsbeispiel eines Verfahrens zur Ermittlung einer Patientenbewegung während einer medizinischen Bildgebungsmessung mit der Magnetresonanzvorrichtung 10 dargestellt. Dabei wird in S120 bei der Ermittlung der Patientenbewegung mit Hilfe der Referenz-Bilddaten in S121 ein Referenz-Modell des Kopfbereichs des Patienten 15 erstellt.

Beispielsweise kann die Erstellung des Referenz-Modell des Kopfbereichs des Patienten 15 in S121 umfassen, dass - wie durch Fig. 4 dargestellt - vor der medizinischen Bildgebungsmessung das Gesicht F des Patienten 15, insbesondere als Tiefenbild, als Referenz-Bilddaten aufgenommen wird. Die Referenz-Bilddaten können beispielsweise mit der Kamera 13 aufgenommen werden, bevor das Oberteil 110 der Kopfspule 100 montiert wird. Die Referenz-Bilddaten können aber auch beispielsweise durch eine andere Kamera erfasst werden, die sich außerhalb des Messbereichs der Magnetresonanzvorrichtung 10 befindet. Aus dem aufgenommenen Referenz-Bilddaten kann ein dreidimensionales Modell des Gesichts F erstellt werden.

In S122 werden Magnetresonanzsignale als medizinische Bildgebungs-Daten und Patienten-Bilddaten eines Teils des Kopfbereichs des Patienten 15 während der medizinischen Bildgebungsmessung erfasst, wobei die Kopfspule 100 den Kopfbereich des Patienten 15 teilweise abdeckt. Die Patienten-Bilddaten können beispielsweise von der Kamera 13 erfasst werden.

Eine teilweise Abdeckung des den Kopfbereichs des Patienten 15 durch die Kopfspule 100 Situation ist beispielhaft in Fig. 6 dargestellt. Hier deckt das Oberteil 110 der Kopfspule 100 in einem Teilbereich AC des Gesichts F des Patienten 15 ab, während in einem Teilbereich AT das Gesicht F des Patienten 15 erfasst wird. Beispielsweise können die Patienten-Bilddaten während der medizinischen Bildgebungsmessung als ein kontinuierliches Tiefenbild, z.B. als Video mit einer Wiederholfrequenz von 60 Hz, von dem Patienten 15 und der Kopfspule von der Kamera 13 aufgekommen werden.

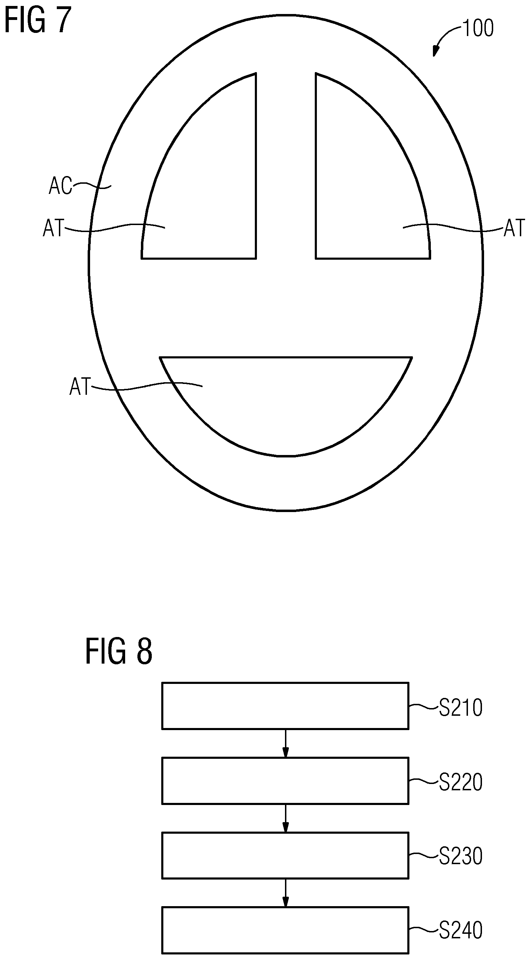

In S123 wird eine Geometrie-Information der Kopfspule 100 bereitgestellt. Dies kann beispielsweise eine Erfassung von Modul-Bilddaten des Moduls der Bildgebungsvorrichtung und/oder ein Abrufen geometrischer Daten des Moduls aus einer Datenbank umfassen. Beispielhaft ist eine Geometrie-Information einer Kopfspule 100 in Fig. 7 dargestellt.

Die Modul-Bilddaten können beispielsweise mit der Kamera 13 aufgenommen werden, wenn der Patient 15 noch nicht in der Kopfspule 100 gelagert ist. Modul-Bilddaten können beispielsweise als Masken-Tiefenbild aufgenommen werden.

Ferner kann die Geometrie-Information als geometrische Daten des Moduls, z.B. als CAD-Daten, vorliegen. Insbesondere ist die Magnetresonanzvorrichtung 10 ausgebildet, einen Typ des Moduls der Magnetresonanzvorrichtung 10 zu erkennen, insbesondere einen Lokalspulentyp bzw. einen Typ der Kopfspule 100. Abhängig vom erkannten Typ des Moduls kann eine entsprechende Geometrie-Information bereitgestellt werden. So können beispielsweise unterschiedliche Modulformen berücksichtigt werden. Die Erkennung des Typs des Moduls kann beispielsweise durch ein Auslesen eines Speichers, z.B. EEPROM, des Moduls erfolgen.

In S124 werden Anpassungsdaten aus den Patienten-Bilddaten mit Hilfe der Geometrie-Information erstellt. Die Geometrie-Information kann dabei insbesondere als Maske dienen. Dabei werden beispielsweise Differenz-Bilddaten aus der Geometrie-Information des Moduls und den Patienten-Bilddaten erstellt. Insbesondere kann ein Masken-Tiefenbild des Moduls, z.B. der Kopfspule 100, von dem kontinuierliches Tiefenbild subtrahiert werden, so dass lediglich Segmente, z.B. Gesichtssegmente, des Patienten 15 übrigbleiben.

Dabei können die Differenz-Bilddaten insbesondere durch Anwenden einer durch maschinelles Lernen trainierten Funktion auf die Geometrie-Information des Moduls und die Patienten-Bilddaten erzeugt werden.

In S125 wird eine Bewegung des Kopfes, insbesondere des Gesichts, des Patienten 15 durch Anpassen der Anpassungsdaten an das Referenz-Modell ermittelt.

Die Ermittlung der Patientenbewegung durch Anpassen der Anpassungsdaten an das Referenz-Modell kann ein Anwenden einer durch maschinelles Lernen trainierten Funktion auf die Anpassungsdaten umfassen, wobei Patientenbewegungsdaten erzeugt werden.

Beispielsweise kann ein Video der übriggebliebenen Segmente des Patienten 15 an das volle Referenz-Modell gefittet werden. Somit kann nur anhand der übriggebliebenen Segmente das gesamte Gesichtsfeld rekonstruiert werden.

In Fig. 8 wird ein computerimplementiertes Verfahren zum Bereitstellen einer trainierten Funktion zur Erzeugung von Differenz-Bilddaten schematisch dargestellt.

In S210 werden Trainings-Modul-Bilddaten und Trainings-Patienten-Bilddaten empfangen. In S220 werden Trainings-Differenz-Bilddaten empfangen, wobei die Trainings-Differenz-Bilddaten mit den Trainings-Modul-Bilddaten und Trainings-Patienten-Bilddaten verknüpft sind.

In S230 wird eine Funktion basierend auf den Trainings-Modul-Bilddaten, den Trainings-Patienten-Bilddaten und den Trainings-Differenz-Bilddaten trainiert. In 240 wird eine trainierte Funktion zur Erzeugung von Differenz-Bilddaten bereitgestellt.

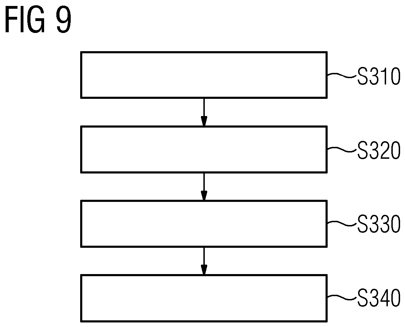

In Fig. 9 wird ein computerimplementiertes Verfahren zum Bereitstellen einer trainierten Funktion zur Erzeugung von Patientenbewegungsdaten schematisch dargestellt.

In S310 werden Trainings-Anpassungsdaten empfangen. In S320 werden Trainings-Patientenbewegungsdaten empfangen, wobei die Trainings-Patientenbewegungsdaten mit den Trainings-Anpassungsdaten verknüpft sind.

In S330 wird eine Funktion basierend auf den Trainings-Patientenbewegungsdaten und den Trainings-Anpassungsdaten trainiert. In S340 wird eine trainierte Funktion zur Erzeugung von Patientenbewegungsdaten bereitgestellt.

Es wird abschließend noch einmal darauf hingewiesen, dass es sich bei den vorhergehend detailliert beschriebenen Verfahren und Vorrichtungen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" oder Begriff "Modul" nicht aus, dass die betreffenden Komponenten aus mehreren zusammenwirkenden TeilKomponenten bestehen, die gegebenenfalls auch räumlich verteilt sein können.

## Patentansprüche

1. Computerimplementiertes Verfahren zur Ermittlung einer Patientenbewegung während einer medizinischen Bildgebungsmessung mit einer Bildgebungsvorrichtung, insbesondere einer Magnetresonanzvorrichtung (10), wobei das Verfahren umfasst:
- Erfassung von Referenz-Bilddaten eines Körperbereichs eines Patienten (15),
- Erstellung eines dreidimensionalen Referenz-Modells des Körperbereichs des Patienten (15) anhand der Referenz-Bilddaten,
- Erfassung von Patienten-Bilddaten eines Teils des Körperbereichs des Patienten (15) während der medizinischen Bildgebungsmessung,
- Erstellung von Anpassungsdaten aus den Patienten-Bilddaten,
- Ermittlung der Patientenbewegung mit Hilfe der Referenz-Bilddaten durch Anpassen der Anpassungsdaten an das dreidimensionale Referenz-Modell.

2. Verfahren nach Anspruch 1,
wobei das Verfahren eine Bereitstellung einer Geometrie-Information eines Moduls der Bildgebungsvorrichtung, insbesondere eine Bereitstellung einer Geometrie-Information einer Lokalspule, umfasst,
wobei das Modul den Körperbereich des Patienten während der Erfassung der Patienten-Bilddaten teilweise abdeckt,
wobei die Ermittlung der Anpassungsdaten mit Hilfe der Geometrie-Information erfolgt.

3. Verfahren nach Anspruch 2,
wobei der Körperbereich des Patienten ein Kopfbereich des Patienten ist,
wobei das Modul der Bildgebungsvorrichtung eine Kopfspule (100) ist.

4. Verfahren nach einem der Ansprüche 2 bis 3,
wobei die Erstellung der Anpassungsdaten aus den Patienten-Bilddaten eine Erstellung von Differenz-Bilddaten aus der Geometrie-Information des Moduls und den Patienten-Bilddaten umfasst.

5. Verfahren nach Anspruch 4,
wobei die Erstellung der Differenz-Bilddaten aus der Geometrie-Information des Moduls und den Patienten-Bilddaten umfasst:
- Anwenden einer durch maschinelles Lernen trainierten Funktion auf die Geometrie-Information des Moduls und die Patienten-Bilddaten, wobei Differenz-Bilddaten erzeugt werden.

6. Verfahren nach einem der Ansprüche 2 bis 5,
wobei die Bereitstellung der Geometrie-Information des Moduls der Bildgebungsvorrichtung umfasst:
- Erfassung von Modul-Bilddaten des Moduls der Bildgebungsvorrichtung und/oder
- Abrufen geometrischer Daten des Moduls aus einer Datenbank.

7. Verfahren nach einem der vorangehenden Ansprüche,
wobei die Ermittlung der Patientenbewegung durch Anpassen der Anpassungsdaten an das Referenz-Modell umfasst:
- Anwenden einer durch maschinelles Lernen trainierten Funktion auf die Anpassungsdaten, wobei Patientenbewegungsdaten erzeugt werden.

8. Verfahren nach Anspruch 1,
wobei der Körperbereich ein Gesicht (F) des Patienten (15) ist,
wobei die Erfassung der Referenz-Bilddaten des Körperbereich des Patienten (15) durch einer Miniaturkamera (130) erfolgt, die in eine Kopfspule (100) angeordnet ist.

9. Verfahren nach einem der vorangehenden Ansprüche,
wobei die Ermittlung der Patientenbewegung innerhalb einer vorbestimmten Zeitspanne erfolgt.

10. Verfahren nach einem der vorangehenden Ansprüche,
wobei während der medizinischen Bildgebungsmessung am Patienten (15), insbesondere am Kopf des Patienten (15), keine externe Markierung angebracht ist.

11. Verfahren nach einem der Ansprüche 5 oder 6,
wobei die Erstellung der Differenz-Bilddaten ein ein computerimplementiertes Verfahren zum Bereitstellen einer trainierten Funktion zur Erzeugung der Differenz-Bilddaten umfasst, umfassend
- Empfangen von Trainings-Modul-Bilddaten und Trainings-Patienten-Bilddaten,
wobei die Trainings-Modul-Bilddaten Daten sind, die ein Modul einer Bildgebungsvorrichtung abbilden,
- Empfangen von Trainings-Differenz-Bilddaten,
wobei die Trainings-Differenz-Bilddaten mit den Trainings-Modul-Bilddaten und Trainings-Patienten-Bilddaten verknüpft sind,
- Trainieren einer Funktion basierend auf den Trainings-Modul-Bilddaten, den Trainings-Patienten-Bilddaten und den Trainings-Differenz-Bilddaten,
- Bereitstellen der trainierten Funktion zur Erzeugung von Differenz-Bilddaten.

12. Verfahren nach Anspruch 7,
umfassend ein computerimplementiertes Verfahren zum Bereitstellen der trainierten Funktion zur Erzeugung der Patientenbewegungsdaten umfassend
- Empfangen von Trainings-Anpassungsdaten,
- Empfangen von Trainings-Patientenbewegungsdaten,
wobei die Trainings-Patientenbewegungsdaten mit den Trainings-Anpassungsdaten verknüpft sind,
- Trainieren einer Funktion basierend auf den Trainings-Patientenbewegungsdaten und den Trainings-Anpassungsdaten,
- Bereitstellen der trainierten Funktion zur Erzeugung von Patientenbewegungsdaten.

13. Bildgebungsvorrichtung, insbesondere Magnetresonanzvorrichtung (10), die ausgebildet ist, ein Verfahren nach einem der Ansprüche 1 bis 11 auszuführen.

14. Computerprogrammprodukt, welches ein Programm umfasst und direkt in einen Speicher einer programmierbaren Systemsteuereinheit einer Bildgebungsvorrichtung ladbar ist, mit Programmmitteln, um ein Verfahren nach einem der Ansprüche 1 bis 10 auszuführen, wenn das Programm in der Systemsteuereinheit der Bildgebungsvorrichtung ausgeführt wird.

## Claims

1. Computer-implemented method for determining a patient movement during a medical imaging measurement with an imaging apparatus, in particular a magnetic resonance apparatus (10), wherein the method comprises:
- acquiring reference image data of a body region of a patient (15),
- producing a three-dimensional reference model of the body region of the patient (15) on the basis of the reference image data,
- acquiring patient image data of a part of the body region of the patient (15) during the medical imaging measurement,
- producing adjustment data from the patient image data,
- determining the patient movement with the aid of the reference image data by adjusting the adjustment data to the three-dimensional reference model.

2. Method according to claim 1,
wherein the method comprises providing an item of geometry information of a module of the imaging apparatus, in particular providing an item of geometry information of a local coil,
wherein the module partially covers the body region of the patient during the acquisition of the patient image data, wherein the determination of the adjustment data is carried out with the aid of the geometry information.

3. Method according to claim 2,
wherein the body region of the patient is a head region of the patient,
wherein the module of the imaging apparatus is a head coil (100).

4. Method according to one of claims 2 to 3,
wherein the production of the adjustment data from the patient image data involves a production of differential image data from the geometry information of the module and the patient image data.

5. Method according to claim 4,
wherein the production of the differential image data from the geometry information of the module and the patient image data comprises:
- applying a function trained by machine learning to the geometry information of the module and the patient image data, wherein differential image data is generated.

6. Method according to one of claims 2 to 5,
wherein the provision of the geometry information of the module of the imaging apparatus comprises:
- acquiring module image data of the module of the imaging apparatus and/or
- recalling geometric data of the module from a database.

7. Method according to one of the preceding claims,
wherein the determination of the patient movement by adjusting the adjustment data to the reference model comprises:
- applying a function trained by machine learning to the adjustment data, wherein patient movement data is generated.

8. Method according to claim 1,
wherein the body region is a face (F) of the patient (15), wherein the acquisition of the reference image data of the body region of the patient (15) takes place by means of a miniature camera (130) arranged in a head coil (100).

9. Method according to one of the preceding claims,
wherein the determination of the patient movement takes place within a predetermined time span.

10. Method according to one of the preceding claims,
wherein no external marker is attached to the patient (15), in particular to the head of the patient (15), during the medical imaging measurement.

11. Method according to one of claims 5 or 6,
wherein the production of the differential image data comprises a computer-implemented method for providing a trained function in order to generate differential image data, comprising
- receiving training module image data and training patient image data,
wherein the training module image data is data which map a module of an imaging apparatus,
- receiving training differential image data,
wherein the training differential image data is linked to the training module image data and training patient image data,
- training a function on the basis of the training module image data, the training patient image data and the training differential image data,
- providing the trained function in order to generate differential image data.

12. Method according to claim 7,
comprising a computer-implemented method for providing a trained function in order to generate patient movement data comprising
- receiving training adjustment data,
- receiving training patient movement data,
wherein the training patient movement data is linked to the training adjustment data,
- training a function on the basis of the training patient movement data and training adjustment data,
- providing the trained function for generating patient movement data.

13. Imaging apparatus, in particular magnetic resonance apparatus (10), which is designed to carry out a method according to one of claims 1 to 11.

14. Computer program product which comprises a program and is directly loadable into a memory store of a programmable system control unit of an imaging apparatus and comprises program means in order to carry out a method according to one of claims 1 to 10 when the program is executed in the system control unit of the imaging apparatus.

## Revendications

1. Procédé mis en œuvre par ordinateur de détermination d'un mouvement de patient pendant une mesure d'imagerie médicale par une installation d'imagerie, en particulier par une installation (10) par résonnance magnétique, dans lequel le procédé comprend :
- saisie de données d'image de référence d'une partie du corps d'un patient (15),
- établissement d'un modèle de référence en trois dimensions de la partie du corps du patient (15) à l'aide des données d'image de référence,
- saisie de données d'image de patient d'une partie de la partie du corps du patient (15) pendant la mesure d'imagerie médicale,
- établissement de données d'adaptation à partir des données d'image de patient,
- détermination du mouvement de patient à l'aide des données d'image de référence en adaptant les données d'adaptation au modèle de référence en trois dimensions.

2. Procédé suivant la revendication 1,
dans lequel le procédé comprend une mise à disposition d'une information de géométrie d'un module de l'installation d'imagerie, en particulier une mise à disposition d'une information de géométrie d'une bobine locale,
dans lequel le module recouvre en partie la partie du corps du patient pendant la saisie des données d'image du patient,
dans lequel la détermination des données d'adaptation s'effectue à l'aide de l'information de géométrie.

3. Procédé suivant la revendication 2,
dans lequel la partie du corps du patient est une partie de la tête du patient,
dans lequel le module de l'installation d'imagerie est une bobine (100) de tête.

4. Procédé suivant la revendication 2 à 3,
dans lequel un établissement des données d'adaptation à partir des données d'image du patient comprend un établissement de données d'image de différences à partir de l'information de géométrie du module et des données d'image du patient.

5. Procédé suivant la revendication 4,
dans lequel l'établissement des données d'image de différences à partir de l'information de géométrie du module et des données d'image du patient comprend :
- appliquer une fonction entraînée par apprentissage automatique à l'information de géométrie du module et aux données d'image du patient, des données d'image de différences étant produites.

6. Procédé suivant l'une des revendications 2 à 5,
dans lequel la mise de l'information de géométrie du module à disposition de l'installation d'imagerie comprend :
- saisie de données d'image de module du module de l'installation d'imagerie et/ou
- appel de données géométriques du module dans une base de données.

7. Procédé suivant l'une des revendications précédentes,
dans lequel la détermination du mouvement de patient par adaptation des données d'adaptation au modèle de référence comprend :
- application d'une fonction entraînée par apprentissage automatique aux données d'adaptation, des données de mouvement de patient étant produites.

8. Procédé suivant la revendication 1,
dans lequel la partie du corps est un visage (F) du patient (15), dans lequel la saisie des données d'image de référence de la partie du corps du patient (15) s'effectue par un appareil (130) photographique miniature, qui est disposé dans une bobine (100) de tête.

9. Procédé suivant l'une des revendications précédentes,
dans lequel la détermination de mouvement de patient s'effectue dans un laps de temps déterminé à l'avance.

10. Procédé suivant l'une des revendications précédentes,
dans lequel, pendant la mesure d'imagerie médicale, il n'est pas apposé de repère extérieur sur le patient (15), en particulier sur la tête du patient (15).

11. Procédé suivant l'une des revendications 5 ou 6,
dans lequel l'établissement des données d'image de référence comprend un procédé mis en œuvre par ordinateur de mise à disposition d'une fonction entraînée de production des données d'image de différences comprenant :
- réception de données d'image de module d'entraînement et de données d'image de patient d'entraînement,
dans lequel les données d'image de module d'entraînement sont des données, qui reproduisent un module d'une installation d'imagerie,
- réception de données d'image de différences d'entraînement, dans lequel les données d'image de différences d'entraînement sont combinées aux données d'image de module d'entraînement et aux données d'image de patient d'entraînement,
- entraînement d'une fonction sur la base des données d'image de module d'entraînement, des données d'image de patient d'entraînement et des données d'image de différences d'entraînement,
- mise à disposition de la fonction entraînée pour la production de données d'image de différences.

12. Procédé suivant la revendication 7,
comprenant un procédé mis en œuvre par ordinateur de mise à disposition de la fonction entraînée pour la production des données de mouvement de patient, comprenant :
- réception de données d'adaptation de données d'entraînement,
- réception de données de mouvement de patient d'entraînement, dans lequel les données les données de mouvement de patient d'entraînement sont combinées aux données d'adaptation d'entraînement,
- entraînement d'une fonction sur la base des données de mouvement de patient d'entraînement et des données d'adaptation d'entraînement,
- mise à disposition de la fonction entraînée pour la production de données de mouvement de patient.

13. Installation d'imagerie, en particulier installation (10) de résonnance magnétique, qui est constituée pour exécuter un procédé suivant l'une des revendications 1 à 11.

14. Produit de programme d'ordinateur, qui comprend un programme et qui peut être chargé directement dans la mémoire d'une unité de commande de système programmable d'une installation d'imagerie, comprenant des moyens de programme pour exécuter un procédé suivant l'une des revendications 1 à 10, lorsque le programme est exécuté dans l'unité de commande de système de l'installation d'imagerie.
